# FASCICULE DE BREVET EUROPEEN

(11) **EP 2 131 811 B1**
(45) Date de publication et mention de la délivrance du brevet: **04.03.2020**
(21) Numéro de dépôt: 08775707.6
(22) Date de dépôt: 11.03.2008
(51) Int. Cl.: A61K 9/00, A61K 9/20, A23K 20/10, A23K 20/28, A23K 20/121, A23K 20/142, A23K 20/163, A23K 50/42, A61K 31/78, A61K 31/198, A61K 33/06, A61K 33/12, A61P 1/12

(54) **PRODUIT APPÉTENT**
SCHMACKHAFTES PRODUKT
PALATABLE PRODUCT

(30) Priorité: 14.03.2007 FR 0753828; 20.03.2007 FR 0702006
(43) Date de publication de la demande: 16.12.2009
(73) Titulaire: Vetinnov, 31520 Ramonville St Agne (FR)
(72) Inventeur: MALOISEL, Jean-Pierre, 31000 Toulouse (FR)
(74) Mandataire: Nony
(86) Numéro de dépôt international: PCT/FR2008/050406
(87) Numéro de publication internationale: WO 2008/129191

(56) Documents cités:
- EP-A- 0 482 580
- EP-A- 1 481 672
- WO-A-03/035027
- WO-A-2004/043427
- WO-A2-2007/090987
- GB-A- 1 337 176
- US-A- 4 055 676

## Description

L'invention concerne un produit appétent destiné à servir comme médicament, comme complément alimentaire ou comme support appétent permettant d'enrober une substance active destinée à un animal, en particulier pour les chiens, les chats, les chevaux, les porcs et les ruminants, ainsi que des procédés permettant la fabrication et l'utilisation dudit produit.

L'administration aux animaux de substances actives par voie orale, en particulier de médicaments, est souvent difficile du fait du mauvais goût de certaines de ces substances et du sens très développé de l'odorat et du goût de ces animaux.

En cas de refus d'absorption, les chances de succès du traitement vétérinaire peuvent chuter dramatiquement.

Il existe donc un besoin pour un conditionnement des substances actives apte à déjouer la vigilance des animaux, en particulier des chiens, des chats, des chevaux, des porcs et des ruminants, et notamment des chats.

On connaît de EP 1 562 549 un support appétent pour une substance active destinée à un animal, notamment pour un médicament. La matrice dudit support comporte au moins une surface collante suffisamment adhésive pour fixer la substance active par simple contact de la substance active avec la surface collante. Il est suggéré d'utiliser une matrice « neutre », c'est-à-dire ne présentant sensiblement aucun goût, de manière qu'une même matrice puisse servir à différents types d'animaux. Cependant aucun mode de réalisation d'un tel support n'est décrit.

Par ailleurs, il existe des médicaments antidiarrhéiques, mais qui peuvent être difficiles à faire ingérer aux animaux, notamment aux chats et aux chiens. En particulier, les argiles de la famille des smectites, le charbon actif et les pectates sont difficiles à faire ingérer aux animaux, et notamment aux chats et aux chiens.

Il existe aussi des médicaments à base de L-Lysine permettant de lutter contre l'infection des chats par l'herpès virus, mais qui peuvent également être difficiles à faire ingérer aux animaux, notamment aux chats.

Il existe donc un besoin pour un produit appétent, pouvant servir comme médicament ou comme complément alimentaire, ou pour envelopper une substance active,
et convenant à différents types d'animaux, en particulier les chiens et les chats, et notamment les chatons. Il existe également un besoin pour un procédé de fabrication dudit produit.

Le but de la présente invention est de fournir un tel produit.

Selon l'invention, on atteint ce but au moyen d'un produit appétent destiné à servir de médicament ou de complément alimentaire, ou à enrober, au moins partiellement, une substance active initialement indépendante du produit ayant une forme pâteuse ou plastique, remarquable en ce qu'il comporte en pourcentages massiques, sur la base de la masse totale du produit,
- une quantité efficace d'un agent thérapeutique incorporé, au moins 5 %, de préférence au moins 15 %, de préférence encore au moins 20 % d'un additif antidiarrhéique, choisi dans le groupe formé par une argile de la famille des smectites, de préférence la montmorillonite, un charbon actif, un pectate, et leur mélanges, et/ou au moins 15 % de L-Lysine,
- 5% à 90 % de protéines d'origine animale,
- de 3 % à 50 % de sirop de glucose, l'équivalent en dextrose (DE) du sirop de glucose étant supérieur à 10 et inférieur à 25, de préférence inférieur ou égal à 15, et,
- optionnellement, de préférence au moins 2 %, de préférence au moins 4 %, de préférence au moins 5 % d'amidon de riz, de préférence hydrolysé.

Une teneur en L-Lysine supérieure à 15 % permet de s'assurer d'une action efficace de la L-Lysine contre l'herpès virus, à la différence notamment de la Lysine naturellement présente dans la viande.

De préférence, le pourcentage massique dudit amidon de riz est inférieur à 20 %, de préférence inférieur à 10 %, de préférence encore inférieur à 7 %.

De préférence, la quantité d'additif antidiarrhéique et/ou de L-Lysine est inférieure à 50 %, de préférence inférieure à 40 %, de préférence inférieure à 30 %, en pourcentages massiques sur la base totale du produit. Une quantité d'anti-diarrhéique et/ou de L-Lysine comprise entre 23 et 27 %, de préférence d'environ 25 % est considérée comme optimale.

Par « substance active initialement indépendante », il faut comprendre que le produit tel que fabriqué ne contient pas la substance active. Par « substance active » il faut comprendre toute substance, autre que l'additif antidiarrhéique et la L-Lysine, pouvant avoir un effet de soin, au sens médical et/ou cosmétique et/ou alimentaire, préparée par exemple, sous forme de comprimés, de tablettes, de gélules, de pilules, ou de poudres.

Comme on le verra plus en détail dans la suite de la description, un produit appétent selon l'invention est bien accepté à la fois par les chats et les chiens et, de manière remarquable, par les chats, et, simultanément, présente avantageusement un effet antidiarrhéique et/ou un effet anti-herpès.

De plus, la présence de l'amidon de riz rend collant le produit. Avantageusement, la substance active y adhère donc efficacement. Associé au sirop de glucose, l'amidon de riz permet de constituer un produit homogène, pâteux et collant, efficace pour faire ingérer des substances actives, à la fois par les chats et par les chiens.

Sauf indication contraire, tous les pourcentages sont des pourcentages massiques, sur la base de la masse totale du produit.

De préférence, le produit présente encore une ou plusieurs des caractéristiques optionnelles suivantes :
- Le produit comporte une farine de viande, de préférence une farine de volaille, le total de ladite farine et de l'agent thérapeutique incorporé et/ou d'un agent non thérapeutique incorporé étant supérieur à 5 %, 20 %, 30 %, 40 % et/ou inférieur à 80 %, 70 %, 60 %, 50 %, voire 45 %, en pourcentages en masse sur la base du produit;
- Le produit comporte un humectant, choisi parmi des alcools ou un mélange de ceux-ci, de préférence parmi le propylène glycol, le glycérol, le sorbitol ou un mélange de ceux-ci. Une quantité d'un mélange de propylène glycol et de glycérol supérieure ou égale à 3 % et/ou inférieure ou égale à 15 % est préférable, la quantité en propylène glycol étant de préférence supérieure ou égale à 3 %, de préférence à 5 %, et/ou inférieure ou égale à 15 %, de préférence à 10 %, la quantité en glycérol étant de préférence supérieure ou égale à 5 %, de préférence à 9 %, et/ou inférieure ou égale à 20 %, de préférence à 15 %. Une quantité de sorbitol supérieure ou égale à 3 %, de préférence à 5 %, et/ou inférieure ou égale à 15 %, de préférence à 10 %, est également préférable. De préférence encore, le produit comporte une quantité de lécithine supérieure ou égale à 1 %, de préférence à 3 %, et/ou inférieure ou égale à 15 %, de préférence à 8 %.

Avantageusement, ces caractéristiques permettent d'obtenir une activité en eau (Aw) du produit inférieure à 0,75 et une durée de conservation atteignant environ 18 mois.

Le produit comporte une quantité de protéines d'origine animale, choisies de préférence dans le groupe formé par les farines de volailles, de porc, de poisson et de leurs mélanges, de préférence supérieure ou égale à 30 %, et/ou inférieure ou égale à 50 %.

Le produit comporte une quantité d'amidon modifié, de préférence d'amidon de riz modifié, de préférence de l'AXFGP (AX Finest Grade Précuit) commercialisé par la société REMY Industries (Belgique) qui est de l'amidon de riz préalablement hydrolysé, de préférence supérieure ou égale à 4 %, et/ou inférieure ou égale à 90 %, de préférence à 50 %, de préférence encore à 20 %.

L'amidon de riz est cuit. De préférence il contient plus de 90 % en poids, de préférence plus de 95 % en poids d'amylopectine.

De préférence, l'amidon de riz est cireux, c'est-à-dire qu'il ne contient sensiblement que de l'amylopectine. Par comparaison, un amidon « régulier » ne contient environ que 80 % d'amylopectine. Avantageusement, la teneur élevée en amylopectine confère un caractère très collant au produit et permet de limiter sa teneur en glucose.

Le produit comporte une quantité de matières grasses, de préférence choisies parmi des huiles, des graisses, des émulsifiants ou un mélange de ceux-ci. Une quantité d'huile, de préférence choisies parmi les huiles végétales, en particulier les huiles de colza et/ou de tournesol, supérieure ou égale à 0,01 %, de préférence à 1 %, et/ou inférieure à 10 %, de préférence à 5 %, est souhaitable. Une quantité de graisse, de préférence d'origine animale, en particulier des graisses de canard et/ou de porc, supérieure ou égale à 0,01 %, et/ou inférieure à 10 %, de préférence à 2 %, est souhaitable. Une quantité d'émulsifiant supérieure ou égale à 0,01 %, et/ou inférieure à 10 %, de préférence à 1 %, est encore souhaitable, l'émulsifiant préféré étant le 2901 commercialisé par la société Brenntag N.V.

Le produit comporte des conservateurs, constitués de préférence de BHA (HydroxyAnisole butylé) et/ou de galate de propyle et/ou d'acide citrique et/ou d'acide lactique et/ou d'acide propénoïque et/ou d'acide sorbique et/ou d'acide phosphorique. Une quantité totale de conservateurs, supérieure ou égale à 0,01 %, et/ou inférieure ou égale à 10 %, de préférence à 3 % est préférée. Les conservateurs préférés sont choisis parmi le Thermox™, le Pet SAVOR®, le sorbate ou un mélange de ceux-ci. Une quantité de Thermox™ supérieure ou égale à 0,01 %, et/ou inférieure ou égale à 2 %, de préférence à 1 %, est souhaitable. Une quantité de Pet SAVOR®, supérieure ou égale à 0,5 %, de préférence à 0,7 %, et/ou inférieure à 5 %, de préférence à 2 %, est également souhaitable. Une quantité de sorbate, de préférence de sorbate de potassium, supérieure ou égale à 0,01 %, de préférence à 0,1 %, et/ou inférieure à 5 % de préférence à 1,5 %, est encore souhaitable.

Le produit comporte une quantité d'un exhausteur d'appétence, de préférence spécifique au type d'animal auquel est destiné le produit et choisi parmi les produits de type Superpremium, de préférence à base de foie de porc, de préférence encore les exhausteurs 8P pour les chiens et 9P ou 9M pour les chats commercialisés par SDF Diana, supérieure ou égale à 1 %, de préférence à 2 % et/ou inférieure ou égale à 15 %, de préférence à 10 %, de préférence à 6 %.

Le produit comporte de 5 à 90 % de protéine d'origine animale, de 4 à 90 % d'amidon, moins de 10 % de matières grasses et de 0,01 % à 10 % d'au moins un conservateur, en pourcentages massiques sur la base de la masse totale du produit.

Le pourcentage massique du sirop de glucose est supérieur à 5 % et/ou inférieur à 20 %, de préférence inférieur à 15 %, de préférence encore inférieur à 10 %, voire inférieur à 7 %, voire même à 6,5 %.

L'équivalent en dextrose (DE) du sirop de glucose est supérieur à 15, de préférence même à 18 et/ou inférieur à 22.

Le produit selon l'invention peut être un médicament, un complément alimentaire ou un support.

On décrit également un procédé de fabrication d'un produit appétent destiné à traiter la diarrhée ou à faciliter l'ingestion, par un animal, notamment un chat, d'une substance active et/ou à lutter contre l'infection par un herpès virus, ce procédé comportant une étape dans laquelle on mélange différents constituants et étant remarquable en ce que lesdits constituants et leurs quantités sont déterminés de manière à obtenir un produit selon l'invention.

L'invention concerne aussi un procédé de conditionnement selon la revendication 12.

De manière plus générale, l'inventeur a constaté qu'un produit appétent selon l'invention masque remarquablement bien le goût, voire l'odeur, des agents thérapeutiques qui y sont incorporés.

Par "agent thérapeutique ou non thérapeutique incorporé", on entend les agents thérapeutiques ou non thérapeutiques qui, notamment lorsqu'ils sont sous la forme d'une poudre, sont dispersés au sein du produit appétent. Autrement dit, les agents thérapeutiques ou non thérapeutiques incorporés sont des agents qui sont mélangés avec les autres constituants du produit au moment de la fabrication de ce dernier. Ils ne doivent donc pas être confondus avec la substance active initialement indépendante, généralement sous la forme de comprimés, de tablettes, de gélules, de pilules ou de poudre que l'utilisateur peut enrober avec le produit appétent pour la conditionner et pouvoir la faire ingérer par un animal.

Par « agent thérapeutique », on désigne toute substance ou composition présentée comme ayant des propriétés curatives ou préventives à l'égard de maladies animales, ainsi que tout produit pouvant être administré à l'animal en vue d'établir un diagnostic médical ou de restaurer, corriger ou modifier ses fonctions organiques.

L'agent thérapeutique peut être un additif antidiarrhéique ou la L-Lysine, mais d'autres agents thérapeutiques sont envisageables, et notamment un agent antibiotique, anti-inflammatoire, antiparasitaire, psychotrope ou assimilé, un vaccin, un régulateur hormonal ou endocrinien. Le produit selon l'invention s'est avéré particulièrement efficace lorsque l'agent thérapeutique est un additif antidiarrhéique ou la L-Lysine.

De manière générale, l'invention concerne également un procédé de conditionnement d'une substance active au sein d'un support appétent, ladite substance active, destinée à un animal, notamment un animal domestique, et en particulier un chat ou un chien, étant initialement indépendante dudit support, procédé dans lequel l'utilisateur enrobe au moins partiellement ladite substance active dans ledit support, remarquable en ce que le support comporte de 3 à 50 % de sirop de glucose, en pourcentage massique sur la base de la masse totale du support, l'équivalent en dextrose du sirop de glucose étant compris entre 5 et 60, et une quantité efficace d'un agent thérapeutique incorporé au sein du support.

Une quantité "efficace" d'un agent thérapeutique est une quantité permettant au produit d'avoir un effet thérapeutique ou prophylactique détectable. Des essais permettent de déterminer, en fonction de l'agent thérapeutique considéré, la teneur minimale à partir de laquelle l'agent thérapeutique est efficace. Par exemple, dans le cas d'un agent additif antidiarrhéique, on considère qu'une quantité efficace est une quantité d'au moins 5 %, en pourcentage en masse sur la base de la masse du support appétent. Dans le cas de la L-Lysine, on considère qu'une quantité efficace est une quantité au moins égale à 15 % en pourcentage en masse sur la base de la masse du support appétent.

Avantageusement, le procédé de conditionnement selon ce mode de réalisation permet une bithérapie. Le support appétent selon l'invention permet ainsi de faciliter l'administration de la substance active initialement indépendante tout en autorisant l'administration d'un agent thérapeutique, par exemple d'un agent thérapeutique dont l'action vient compléter celle de la substance active initialement indépendante et/ou d'un agent thérapeutique difficile à faire ingérer sous une autre forme.

Le traitement de l'animal en est avantageusement facilité.

Ce support appétent peut notamment être un produit appétent selon l'invention et présenter une ou plusieurs des caractéristiques optionnelles de ce produit.

On décrit également un ensemble thérapeutique comportant :
- un support appétent comportant, en pourcentage massique sur la base de la masse totale du support, de 3 à 50 % de sirop de glucose, l'équivalent en dextrose du sirop de glucose étant compris entre 5 et 60 et une quantité efficace d'un agent thérapeutique incorporé au sein du support, et
- une substance active sous la forme d'une pilule, d'une tablette, d'une gélule, d'un comprimé ou d'une poudre enrobée, au moins partiellement, par ledit support.

Ce support appétent peut notamment être un produit appétent selon l'invention et présenter une ou plusieurs des caractéristiques optionnelles de ce produit.

Une poudre est dite « enrobée » dans un support appétent selon l'invention lorsqu'elle n'est pas dispersée au sein de ce support, c'est-à-dire qu'elle n'est pas distribuée à travers la masse de ce support. Typiquement, une poudre versée sur un support déchiré qui a ensuite été reconstitué de manière à ne plus exposer la poudre vers l'extérieur est une poudre enrobée (et non incorporée au sein du support).

L'efficacité d'un produit appétent selon l'invention à déjouer la vigilance des animaux permet également d'envisager de l'utiliser pour faire ingérer à un animal, à la place ou en complément de l'agent thérapeutique, un agent non thérapeutique, par exemple des acides gras polyinsaturés, notamment de type oméga 3 ou oméga 6, ou une huile essentielle.

On décrit également un procédé de préparation d'une nourriture pour un animal, notamment un chat ou un chien, selon lequel on incorpore dans ladite nourriture un complément alimentaire selon l'invention. On décrit aussi la nourriture ainsi préparée.

On décrit enfin un procédé de traitement ou de prévention de la diarrhée et/ou de l'herpès chez un animal, en particulier chez un chat ou un chien, remarquable en ce qu'on administre audit animal un produit selon l'invention.

En particulier, on décrit un procédé de lutte contre l'infection du chat par l'Herpes virus, remarquable en ce qu'on administre audit animal un produit appétent selon l'invention comportant au moins 15 % de L-Lysine, en pourcentage massique sur la base du produit.

D'autres caractéristiques et avantages de la présente invention apparaîtront à la lecture de la description qui va suivre et à l'examen du dessin annexé dans lequel :
- la figure 1 représente une vue en coupe transversale d'un produit selon l'invention,
- la figure 2 représente une vue en coupe transversale du produit représenté sur la figure 1, après enrobage d'un comprimé par le produit.

Sur les différentes figures, des références identiques ont été utilisées pour désigner des objets identiques ou analogues.

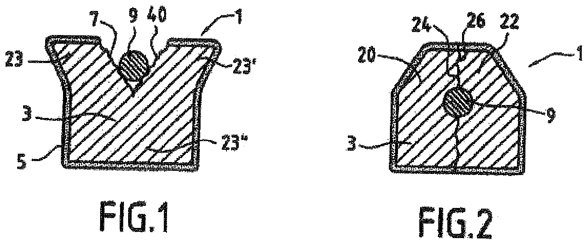

La figure 1 représente un produit 1 comportant une matrice 3 recouverte au moins en partie d'une couche 5 en un matériau appétent ou « exhausteur d'appétence ». Le produit 1 est fourni sous la forme d'un cube sensiblement homogène que l'utilisateur déchire en deux morceaux 20 et 22.

Sur la figure 1, le produit 1 est représenté dans un état intermédiaire entre son état initial sensiblement cubique et son état déchiré en deux morceaux 20 et 22. La matrice 3 comporte, dans cet état intermédiaire, deux extrémités 23 et 23' reliées par une base commune 23". La matrice 3, une fois déchirée expose une surface de contact 27 formée des surfaces 29 et 28 des morceaux 20 et 22, respectivement, délimitant la déchirure.

Avantageusement, la couche 5 d'exhausteur d'appétence et la forme massive du cube protègent la matrice 3 du dessèchement. La durée de vie du produit 1 en est augmentée.

La matrice 3 est de préférence une matière à déformation plastique, c'est-à-dire qu'elle conserve sensiblement la forme qui lui est donnée.

La composition de matrice 3 est comme décrite précédemment.

Une composition préférée entre toutes du produit 1 est la suivante, avec en pourcentage massique, sur la base de la masse totale du produit :
- entre 41,5 % et 42,5 %, de préférence environ 41,9 % pour le total d'un agent thérapeutique incorporé, notamment d'un agent antidiarrhéique et/ou de L-Lysine, et/ou d'un agent non-thérapeutique incorporé, et/ou d'au moins une protéine d'origine animale choisie dans le groupe formé par les farines de volaille, de porc, de poissons et de leur mélanges, de préférence une farine de volaille ;
- entre 4 % et 5 % d'un exhausteur d'appétence, de préférence du type Superpremium commercialisé par SDF Diana, de préférence environ 4,5 % ;
- entre 5,3 % et 6,3 % d'amidon de riz hydrolysé, de préférence du type AXFGP, de préférence environ 5,8 % ;
- entre 0 % et 1 % de chlorure de sodium, de préférence environ 0,7 % ;
- entre 0,01 % et 1 % de sorbate, de préférence environ 0,7 % ;
- entre 0,01 % et 0,1 % de Thermox™, de préférence environ 0,08 % ;
- entre 11 % et 12 % de glycérol, de préférence environ 11,5 % ;
- entre 7 % et 8 % de propylène glycol ou de sorbitol, de préférence environ 7,7 % ;
- entre 5,5 % et 6,5 % de sirop de glucose avec un équivalent en dextrose (D.E.) compris entre 8 et 22, de préférence environ 5,8 % de sirop de glucose avec un équivalent en dextrose (D.E.) d'environ 20 ;
- entre 2 % et 3 % d'huile, de préférence de tournesol et/ou de colza, de préférence environ 2,6 % ;
- entre 0,01 % et 1 % de graisses, de préférence de canard et/ou de porc, de préférence environ 0,6 % ;
- entre 0,6 et 1,6 % de Pet SAVOR®, de préférence environ 1,2 % ;
- entre 5,2 % et 6,2 % de lécithine, de préférence de soja, de préférence environ 5,7 % ;
- entre 10,3 % et 11,3 % d'eau, de préférence environ 10,8 % ;
- entre 0,01 % et 1 % d'émulsifiant, de préférence environ 0,4 %.

De manière surprenante, cette matrice est bien acceptée à la fois par les chiens et par les chats. Elle est plastique et présente un caractère collant qui permet une agrégation des morceaux 20 et 22 par simple pression à la main. Le caractère collant permet la fixation du comprimé 9.

Les agents thérapeutiques ou non-thérapeutiques incorporés peuvent notamment être ceux cités précédemment.

L'exhausteur d'appétence, de préférence le Superpremium™, peut être présente à la surface de la matrice 3 sous forme d'une couche 5 ou bien être contenu directement au sein de la matrice 3.

Un procédé préféré de fabrication d'un produit selon l'invention comporte les différentes étapes suivantes :
a) mélange des constituants se présentant sous forme de poudres, en particulier du sirop de glucose lorsque celui-ci est sous forme de poudre (s'il est sous forme liquide il sera alors incorporé à l'étape b)), et/ou des protéines d'origine animale et/ou de l'amidon et/ou du sorbate et/ou du Thermox™ et/ou du sel, de façon à obtenir une poudre homogène,
b) b) mélange des composés liquides et/ou pâteux, en particulier d'un humectant et/ou des matières grasses, et/ou d'un conservateur et/ou de la lécithine et/ ou de l'eau, avec ladite poudre homogène,
c) c) repos à froid, dans des conditions bien connues de l'homme du métier pour faciliter le passage en machine, suivi d'une extrusion toujours à froid, en particulier au moyen d'un extrudeur mono vis de type « machine à pâtes » ou poussoir, de manière à obtenir une matrice,
d) d) optionnellement dépôt de l'exhausteur d'appétence sur cette matrice, de manière à former une couche continue ou, de préférence discontinue. Il existe dans ce dernier cas des zones de la matrice non couvertes par l'exhausteur de manière à permettre une manipulation du produit sans contact des doigts avec l'exhausteur de goût. Ce dernier peut en effet dégager une odeur désagréable pour l'homme.

Le produit obtenu peut alors être emballé, de préférence sous forme de cubes emballés individuellement, afin de limiter leur dessèchement et faciliter leur manipulation. Le produit recouvert de l'exhausteur d'appétence peut être préalablement fendu, percé, ou rainuré de manière à prédéfinir les futures surfaces de contact avec la substance active. L'exhausteur d'appétence utilisé à l'étape d) est de préférence spécifique à l'animal auquel le produit est destiné.

Cependant, selon l'invention, aux étapes a) et b), on choisit une proportion entre 3 % et 50 % de syrop de glucose avec une DE comprise entre 5 et 60, de manière à obtenir en fin d'étape c) une matrice convenant à la fois aux chats et aux chiens. Les coûts de production en sont avantageusement réduits. L'exhausteur d'appétence peut être introduit pendant les étapes a) ou/et b), l'ensemble des autres constituants étant acceptés par les chats et les chiens, les coûts de fabrication restent avantageusement réduits. On notera également que le procédé de fabrication du produit selon l'invention permet de s'affranchir de toute étape de cuisson lors de la fabrication. De préférence, le procédé de fabrication selon l'invention ne contient pas d'étape de cuisson entre les étapes a) et d) incluses.

Les différentes étapes ainsi que l'ordre dans lequel elles sont décrites sont fournis à titre d'exemple illustratif. En particulier, on peut imaginer que les étapes a) et b) soient inversées. De même on peut très bien imaginer que l'exhausteur d'appétence soit introduit au sein de la matrice au cours d'une des étapes a) ou b).

Pour conditionner un comprimé 9 au moyen d'un produit 1 selon l'invention tel que celui représenté sur la figure 1, un utilisateur peut procéder de la manière suivante.

L'utilisateur déchire le produit 1 en deux morceaux 20 et 22. En ouvrant la matrice, l'utilisateur expose les surfaces 28 et 29.

Il positionne ensuite le comprimé 9 sur une des surfaces 28 et 29, et dispose les morceaux 20 et 22 du produit 1 déchiré de manière que les surfaces 28 et 29 soient sensiblement l'une en face de l'autre. Il exerce alors une action de manière à presser l'un contre l'autre ces morceaux de façon à les agréger. Il est aussi possible de déformer la matrice à la main pour augmenter la surface de contact entre le comprimé 9 et les surfaces de contact 28 et 29.

De préférence, la surface de contact couvre sensiblement toute la surface extérieure du comprimé 9 de manière à englober ce dernier.

Les surfaces 28 et 29 du produit selon l'invention sont telles qu'après qu'elles ont été pressées l'une contre l'autre, elles ne se détachent plus spontanément l'une de l'autre. Par « spontanément », on entend « sous le seul effet de l'élasticité de la matrice », c'est-à-dire sans intervention de l'utilisateur.

De préférence, la déchirure est suffisamment large pour permettre l'insertion complète du comprimé 9. Avantageusement, le produit 1 selon l'invention permet ainsi d'encapsuler, d'enrober ou d'englober totalement le comprimé 9 après reconstitution du cube initial. Aucune odeur répulsive ou aucun goût désagréable ne peut donc plus se dégager du comprimé 9 inséré dans le produit 1.

Comme cela apparaît clairement, le conditionnement d'une substance active au moyen d'un produit selon l'invention est très simple et peut être mis en œuvre extemporanément, à l'unité, par le particulier lui-même. Il ne nécessite aucun appareillage.

Avantageusement, le même procédé de conditionnement et les mêmes produits 1 peuvent être utilisés pour des substances actives de formes très différentes.

Avantageusement, l'utilisateur peut conditionner plusieurs substances actives, se présentant éventuellement sous des formes différentes (gélule, comprimé, tablette, poudre etc.) au moyen d'un même produit 1.

L'efficacité d'un produit selon l'invention à déjouer la vigilance des animaux a été testée pour des chiens et des chats. Les produits testés ont la composition suivante :
- entre 41 % et 42 % pour le total de la farine de volailles et de l'additif antidiarrhéique et/ou de la L-Lysine ;
- entre 4,5 % d'exhausteur d'appétence de type Superpremium;
- environ 5,8 % d'AXFGP ;
- environ 0,64 % de chlorure de sodium ;
- environ 0,64 % de sorbate ;
- environ 0,07 % de Thermox™ ;
- environ 11,5 % de glycérol ;
- environ 7,67 % de propylène glycol ;
- entre 5,7 % et 5,85 % de sirop de glucose avec un équivalent en dextrose (D.E.) de 20 ;
- environ 2,57 % d'un mélange d'huile, de graisses et de l'émulsifiant 2901 de Brenntag ;
- environ 1,24 % de Pet SAVOR® ;
- environ 5,76 % de lécithine de soja ;
- environ 10,8 % d'eau.

Une première série d'essais a été effectuée en incorporant 28,5 % de smectite comme additif antidiarrhéique, sur des chats et des chiens. Une deuxième série d'essais a été effectuée avec des chats, en incorporant 25 % de L-Lysine.

L'exhausteur d'appétence est non seulement inclus dans le mélange des poudres mais aussi déposé à l'extérieur de la matrice 3 de façon à former une couche 5 autour du produit. Le produit est alors non collant, y compris après déchirement en plusieurs morceaux. En outre, la périphérie du produit est particulièrement appétente, ce qui est particulièrement avantageux quand l'animal vient renifler le produit.

Les tests sont menés dans les mêmes conditions pour les chiens et pour les chats. On présente deux fois par jour pendant 5 jours à l'animal un comprimé enrobé dans le produit. A chaque prise, l'animal peut soit ne manger ni le produit ni le comprimé, soit manger le produit mais recracher le comprimé soit manger le produit et le comprimé.

Par la suite, les « échecs du produit » représentent le pourcentage des cas où le produit est mangé mais le comprimé recraché, de manière similaire l'efficacité correspond au pourcentage de cas où le produit et le comprimé sont mangés. Les résultats des tests sont résumés dans le tableau 1 suivant :

**TABLEAU 1**

| Agent thérapeutique incorporé | Additif antidiarrhéique | | L-Lysine |
|---|---|---|---|
| | % pour un chien | % pour un chat | % pour un chat |
| Echecs du produit | 2,5 % | 9,5 % | 5 % |
| Efficacité | 90% | 80% | 95 % |

Comme on peut le constater, l'invention fournit un produit très efficace, à la fois pour les chiens et pour les chats. Avantageusement, ce produit peut être utilisé comme médicament, comme complément alimentaire ou comme support pour une substance active initialement indépendante.

Le mode de réalisation décrit et représenté est fourni à titre d'exemple illustratif En particulier la forme et la taille de la matrice, la présence et la nature de l'exhausteur d'appétence, la présence d'une fente, d'une rainure, d'une incision, d'un trou, ou d'une saignée sont optionnelles.

De plus, les caractéristiques préférées mentionnées ci-dessus dans le cadre de la description des figures ou des essais, où le produit selon l'invention est utilisé comme support, sont également préférées lorsque le produit selon l'invention est utilisé comme médicament antidiarrhéique ou à visée anti-herpès, ou comme complément alimentaire, sans être associé à une substance active initialement indépendante.

## Revendications

1. Produit appétent pour un chat ou un chien, destiné à servir comme médicament, comme complément alimentaire ou comme support appétent pour enrober, au moins partiellement, une substance active initialement indépendante du support, ayant une forme pâteuse ou plastique et comportant en pourcentages massiques, sur la base de la masse totale du produit, 5 % à 90% de protéines d'origine animale, 3 % à 50 % de sirop de glucose, l'équivalent en dextrose du sirop de glucose étant supérieur à 10 et inférieur à 25, et:
- au moins 5 % d'un additif anti-diarrhéique choisi dans le groupe formé par une argile de la famille des smectites, un charbon actif, un pectate et leurs mélanges, et/ou
- au moins 15 % de L-Lysine.

2. Produit selon la revendication précédente, comportant au moins 15 % et moins de 50 % dudit additif antidiarrhéique et/ou moins de 50 % de L-Lysine.

3. Produit selon la revendication précédente, comportant au moins 20 % et moins de 30 % dudit additif antidiarrhéique et/ou de L-Lysine.

4. Produit selon l'une quelconque des revendications précédentes, l'additif antidiarrhéique étant de la montmorillonite.

5. Produit selon l'une quelconque des revendications précédentes, comportant au moins 2 % et moins de 7 % d'amidon de riz.

6. Produit selon la revendication précédente, dans lequel l'amidon de riz est cireux et préalablement hydrolysé.

7. Produit selon l'une quelconque des revendications 5 à 6, dans lequel l'amidon de riz comporte plus de 90 % en poids d'amylopectine.

8. Produit selon l'une quelconque des revendications précédentes, **caractérisé en ce que** le pourcentage massique sur la base de la masse totale du produit, du sirop de glucose est supérieur à 5 % et inférieur à 10 %, l'équivalent en dextrose étant supérieur à 15.

9. Produit selon l'une quelconque des revendications précédentes, **caractérisé en ce qu'**il comporte en pourcentages massiques sur la base de la masse totale du produit :
- entre 3 % et 15 % d'un mélange de propylène glycol et de glycérol, les teneurs en propylène glycol et en glycérol étant comprises entre 3 % et 15 % et 5 % à 20 % respectivement,
- entre 1 % et 15 % de lécithine,
- moins de 15 % d'un exhausteur d'appétence.

10. Produit selon l'une quelconque des revendications précédentes, **caractérisé en ce qu'**il comporte en pourcentage massique sur la base de la masse totale du produit :
- de 4 % à 90 % d'amidon,
- de moins de 10 % de matières grasses,
- de 0,01 % à 10 % d'au moins un conservateur.

11. Ensemble comportant un produit appétent selon l'une quelconque des revendications précédentes et une substance active sous la forme d'une pilule, d'une tablette, d'une gélule, d'un comprimé ou d'une poudre, ladite substance active étant, au moins partiellement, enrobée par ledit produit appétent, la substance active, lorsqu'elle est sous la forme d'une poudre, n'étant pas dispersée au sein dudit produit appétent.

12. Procédé de conditionnement d'une substance active au sein d'un support appétent au sein duquel un agent thérapeutique est dispersé en une quantité efficace, ladite substance active, destinée à un animal, étant initialement indépendante dudit support et sous forme de pilule, tablette, gélule ou comprimé, procédé dans lequel l'utilisateur enrobe au moins partiellement ladite substance active dans ledit support, **caractérisé en ce que** le support comporte, en pourcentages massiques, sur la base de la masse totale du produit, de 3 à 50 % de sirop de glucose, l'équivalent en dextrose du sirop de glucose étant compris entre 10 et 25, l'agent thérapeutique étant un additif anti-diarrhéique choisi dans le groupe formé par une argile de la famille des smectites, un charbon actif, un pectate et leurs mélanges, et/ou la L-Lysine.

13. Procédé de conditionnement selon la revendication précédente, **caractérisé en ce que** le support est un produit conforme à l'une quelconque des revendications 1 à 11.

## Patentansprüche

1. Schmackhaftes Produkt für eine Katze oder einen Hund zur Verwendung als Medikament, als Nahrungsergänzungsmittel oder als schmackhafter Träger, um einen anfänglich vom Träger unabhängigen Wirkstoff zumindest teilweise einzuhüllen, mit pastöser oder plastischer Form und enthaltend, in Massenprozent bezogen auf die Gesamtmasse des Produkts, 5% bis 90% Proteine tierischen Ursprungs, 3% bis 50% Glucosesirup, wobei das Dextroseäquivalent des Glucosesirups größer als 10 und kleiner als 25 ist, und
- mindestens 5% eines Anti-Diarrhö-Additivs, ausgewählt aus der Gruppe, die von einem Ton aus der Familie der Smektite, einer Aktivkohle, einem Pektat und deren Gemischen gebildet wird, und/oder
- mindestens 15% L-Lysin.

2. Produkt nach dem vorhergehenden Anspruch, das mindestens 15% und weniger als 50% des Anti-Diarrhoe-Additivs und/oder weniger als 50% L-Lysin enthält.

3. Produkt nach dem vorhergehenden Anspruch, das mindestens 20% und weniger als 30% des Anti-Diarrhoe-Additivs und/oder von L-Lysin enthält.

4. Produkt nach einem der vorhergehenden Ansprüche, wobei das Anti-Diarrhoe-Additiv Montmorillonit ist.

5. Produkt nach einem der vorhergehenden Ansprüche, das mindestens 2% und weniger als 7% Reisstärke enthält.

6. Produkt nach dem vorhergehenden Anspruch, wobei die Reisstärke wachsartig und zuvor hydrolysiert worden ist.

7. Produkt nach einem der Ansprüche 5 bis 6, wobei die Reisstärke mehr als 90 Gew.-% Amylopektin enthält.

8. Produkt nach einem der vorhergehenden Ansprüche, **dadurch gekennzeichnet, dass** der Massenprozentsatz des Glucosesirups, bezogen auf die Gesamtmasse des Produkts, mehr als 5% und weniger als 10% beträgt, wobei das Dextroseäquivalent größer als 15 ist.

9. Produkt nach einem der vorhergehenden Ansprüche, **dadurch gekennzeichnet, dass** es, in Massenprozent bezogen auf die Gesamtmasse des Produkts, Folgendes enthält:
- zwischen 3% und 15% eines Gemischs von Propylenglykol und Glycerin, wobei die Gehalte an Propylenglykol und Glycerin zwischen 3% und 15% bzw. von 5% bis 20% liegen,
- zwischen 1% und 15% Lecithin,
- weniger als 15% eines Geschmacksverstärkers.

10. Produkt nach einem der vorhergehenden Ansprüche, **dadurch gekennzeichnet, dass** es, in Massenprozent bezogen auf die Gesamtmasse des Produkts, Folgendes enthält:
- von 4% bis 90% Stärke,
- weniger als 10% Fette,
- von 0,01% bis 10% mindestens eines Konservierungsmittels.

11. Zusammenstellung, enthaltend ein schmackhaftes Produkt nach einem der vorhergehenden Ansprüche und einen Wirkstoff in Form einer Pille, eines Täfelchens, einer Kapsel, einer Tablette oder eines Pulvers, wobei der Wirkstoff zumindest teilweise von dem schmackhaften Produkt eingehüllt ist, wobei der Wirkstoff, wenn er in Form eines Pulvers vorliegt, nicht in dem schmackhaften Produkt dispergiert ist.

12. Verfahren zur Verpackung eines Wirkstoffs im Inneren eines schmackhaften Trägers, in dem ein therapeutisches Mittel in einer wirksamen Menge dispergiert ist, wobei der für ein Tier bestimmte Wirkstoff anfänglich unabhängig von dem Träger ist und in Form einer Pille, eines Täfelchens, einer Kapsel oder einer Tablette vorliegt, wobei bei dem Verfahren der Anwender den Wirkstoff zumindest teilweise in den Träger einhüllt, **dadurch gekennzeichnet, dass** der Träger, in Massenprozent bezogen auf die Gesamtmasse des Produkts, von 3% bis 50% Glucosesirup enthält, wobei das Dextroseäquivalent des Glucosesirups zwischen 10 und 25 liegt, wobei das therapeutische Mittel ein Anti-Diarrhö-Additiv, ausgewählt aus der Gruppe, die von einem Ton aus der Familie der Smektite, einer Aktivkohle, einem Pektat und deren Gemischen gebildet wird, und/oder L-Lysin ist.

13. Verpackungsverfahren nach dem vorhergehenden Anspruch, **dadurch gekennzeichnet, dass** der Träger ein Produkt nach einem der Ansprüche 1 bis 11 ist.

## Claims

1. Palatable product for a cat or a dog, intended to serve as a medicament, as a food supplement or as a palatable support for at least partially coating an active substance initially independent of the support, having a pasty or plastic form and comprising, as weight percentages, on the basis of the total weight of the product, 5% to 90% of proteins of animal origin, 3% to 50% of glucose syrup, the dextrose equivalent of the glucose syrup being greater than 10 and less than 25, and:
- at least 5% of an anti-diarrhoea additive chosen from the group formed by a clay of the smectite family, an active carbon, a pectate and mixtures thereof, and/or
- at least 15% of L-lysine.

2. Product according to the preceding claim, comprising at least 15% and less than 50% of said anti-diarrhoea additive and/or less than 50% of L-lysine.

3. Product according to the preceding claim, comprising at least 20% and less than 30% of said anti-diarrhoea additive and/or of L-lysine.

4. Product according to any one of preceding claims, the anti-diarrhoea additive being montmorillonite.

5. Product according to any one of the preceding claims, comprising at least 2% and less than 7% of rice starch.

6. Product according to the preceding claim, in which the rice starch is waxy and pre-hydrolysed.

7. Product according to either one of Claims 5 and 6, in which the rice starch comprises more than 90% by weight of amylopectin.

8. Product according to any one of the preceding claims, **characterized in that** the weight percentage, on the basis of the total weight of the product, of the glucose syrup is greater than 5% and less than 10%, the dextrose equivalent being greater than 15.

9. Product according to any one of the preceding claims, **characterized in that** it comprises as weight percentages, on the basis of the total weight of the product:
- between 3% and 15% of a mixture of propylene glycol and of glycerol, the propylene glycol and glycerol contents being between 3% and 15% and 5% and 20%, respectively,
- between 1% and 15% of lecithin,
- less than 15% of a palatability enhancer.

10. Product according to any one of the preceding claims, **characterized in that** it comprises as weight percentage, on the base of the total weight of the product:
- from 4% to 90% of starch,
- less than 10% of fats,
- from 0.01% to 10% of at least one preservative.

11. Assembly comprising a palatable product according to any one of the preceding claims and an active substance in the form of a pill, a lozenge, a gel capsule, a tablet or a powder, said active substance being at least partially coated with said palatable product, the active substance, when it is in the form of a powder, not being dispersed within said palatable product.

12. Method for packaging an active substance within a palatable support within which a therapeutic agent is dispersed in an effective amount, said active substance, intended for an animal, being initially independent of said support and in the form of a pill, lozenge, gel capsule or tablet, in which method the user at least partially coats said active substance in said support, **characterized in that** the support comprises, as weight percentages, on the basis of the total weight of the product, from 3% to 50% of glucose syrup, the dextrose equivalent of the glucose syrup being between 10 and 25, the therapeutic agent being an anti-diarrhoea additive chosen from the group formed by a clay of the smectite family, an active carbon, a pectate and mixtures thereof, and/or L-lysine.

13. Packaging method according to the preceding claim, **characterized in that** the support is a product in accordance with any one of Claims 1 to 11.
